# EUROPEAN PATENT APPLICATION

(11) **EP 4 671 262 A1**
(43) Date of publication of application: **31.12.2025**
(21) Application number: 24760636.1
(22) Date of filing: 14.02.2024
(51) Int. Cl.: C07K 7/06, A61K 8/64, A61K 8/60, A61Q 19/00, A61Q 19/08, A61Q 19/02

(54) **NOVEL PEPTIDE EXHIBITING SKIN CONDITION IMPROVEMENT ACTIVITY AND COSMETIC COMPOSITION CONTAINING SAME**

(30) Priority: 21.02.2023 KR 20230022569
(71) Applicant: Daebong LS Co., Ltd., Incheon 21697 (KR)
(72) Inventor: YEOM, Hyun-Sook, Jeju-si, Jeju-do 63293 (KR); LEE, Chan-Hyung, Chungju-si, Chungcheongbuk-do 27369 (KR); LEE, Hye-Ja, Seogwipo-si, Jeju-do 63571 (KR); HAM, Kyung-Man, Suwon-si, Gyeonggi-do 16533 (KR); PARK, Eun-Ju, Hwaseong-si, Gyeonggi-do 18425 (KR); PARK, Jin-Oh, Seoul 06276 (KR)
(74) Representative: Chas. Hude A/S
(86) International application number: PCT/KR2024/095146
(87) International publication number: WO 2024/177448

(57) **Abstract**

The present invention relates to a novel peptide exhibiting skin condition-improving activity and to a cosmetic composition comprising the peptide, which shows excellent moisturizing, wrinkle-improving and whitening effects.

## Description

### Technical Field

The present invention relates to a novel peptide exhibiting skin condition improving activity and a cosmetic composition comprising the same, which provides excellent effects in soothing the skin, moisturizing, and improving wrinkles.

This application claims priority to Korean Patent Application No. 10-2023-0022569, filed on February 21, 2023, the entire disclosure of which, including the specification and drawings, is incorporated herein by reference.

### Background Art

The skin serves as the body's primary barrier, protecting internal organs from external environmental stimuli such as temperature and humidity changes, ultraviolet (UV) rays, and pollutants. It also plays a crucial role in maintaining homeostasis, including body temperature regulation. However, when various cell-damaging stimuli-such as excessive physical and chemical irritants, stress, nutritional deficiencies, UV radiation, environmental pollutants, and abrupt temperature changes-are applied externally to skin cells, they can induce a range of biochemical changes within the cells. These changes accelerate skin aging, which is manifested through visible signs such as melasma, freckles, blemishes, loss of elasticity, keratinization, and wrinkle formation.

Aging of the skin leads to wrinkle formation, with key contributing factors including exposure to UV radiation and reduced collagen biosynthesis. Skin aging is generally classified into intrinsic aging, which is determined by genetic factors, and extrinsic aging, which is caused by environmental factors such as sunlight. Among these, extrinsic aging is known to be preventable, treatable, or delayable through mechanisms such as the removal of reactive oxygen species (ROS), proliferation of fibroblasts, and promotion of collagen biosynthesis.

Against this technical background, various studies are being conducted on peptides and other substances that can improve skin condition.

### [Prior Art Document]

### [Patent Document]

(Patent Document 0001) Korean Patent Publication No. 10-2022-0156701 (2022.11.28)

### Disclosure of Invention

### Technical Problem

As a result of research and efforts to develop a peptide material that can be used in cosmetic compositions, the present inventors discovered that a novel synthetic peptide exhibits excellent moisturizing, wrinkle-improving, and whitening effects, thereby completing the present invention.

Accordingly, the present invention aims to provide a novel peptide and a cosmetic composition for moisturizing, wrinkle improvement, and whitening comprising the same.

### Solution to Problem

The present invention provides an isolated peptide represented by the amino acid sequence of the following sequence number:
Sequence No. 1: AAQPR

The present invention also provides a polynucleotide encoding the peptide.

In another aspect, the present invention provides a cosmetic composition comprising the peptide or the polynucleotide.

The cosmetic composition may be used for moisturizing, wrinkle improvement, and whitening.

### Advantageous Effects of Invention

The novel peptide of the present invention exhibits excellent moisturizing, wrinkle-improving and whitening effects, and has a short amino acid sequence that allows easy absorption, and causes minimal side effects, making it widely applicable in cosmetic compositions for improving skin condition.

### Brief Description of Drawings

Fig. 1 is the molecular weight analysis result of the peptide prepared in the Examples.
Fig. 2 is a graph showing the intracellular hyaluronic acid production according to the treatment of the peptide prepared in the Examples.
Fig. 3 is a graph showing the intracellular type I procollagen peptide production according to the treatment of the peptide prepared in the Examples.
Fig. 4 is a graph showing the intracellular melanin production according to the treatment of the peptide prepared in the Examples.

### Mode for Carrying out the Invention

Hereinafter, the present invention will be described in detail. Prior to this, the terms and words used in the present specification and claims should not be construed as being limited to their conventional or dictionary meanings, but should be interpreted according to the principle that the inventors are entitled to define the concept of the terms appropriately to best explain their invention. Therefore, the embodiments described in the present specification are merely the most preferred embodiments of the invention and do not represent all the technical ideas of the invention, and it should be understood that various equivalents and modifications that can replace these may exist at the time of filing.

The novel peptide of the present invention may comprise the sequence of SEQ ID NO: 1 as follows:
SEQ ID NO: 1: AAQPR

The amino acids mentioned in this specification are abbreviated according to the IUPAC-IUB nomenclature as follows:
A: Alanin (Ala)
Q: Glutamine (Gln)
P: Proline (Pro)
R: Arginine (Arg)

In the present invention, the term "peptide or a fragment thereof" refers to a polymer composed of two or more amino acids linked by an amide bond (or peptide bond) .

The peptide may have a protecting group bound to the N- or C-terminus in order to obtain chemical stability, enhanced pharmacological properties (such as half-life, absorbability, potency, efficacy, etc.), altered specificity (e.g., broader biological activity spectrum), or reduced antigenicity. In one embodiment, the N-terminus of the peptide may be bound to a protecting group selected from the group consisting of an acetyl group, fluorenylmethoxycarbonyl group, formyl group, palmitoyl group, myristyl group, stearyl group, butoxycarbonyl group, allyloxycarbonyl group, and polyethylene glycol (PEG); and/or the C-terminus of the peptide may be bound to a protecting group selected from the group consisting of an amino group (-NH₂), tertiary alkyl group, and azide (-NHNH₂). In addition, the peptide may optionally further comprise a targeting sequence, a tag, a labeled residue, or an amino acid sequence specifically designed for a particular purpose to increase the half-life or stability of the peptide.

The peptide of the present invention can be synthesized by methods well known in the art, for example, using an automated peptide synthesizer, or can be produced by genetic engineering techniques, but is not limited thereto.

The present invention also provides a polynucleotide encoding the peptide. By inserting the polynucleotide encoding the peptide into a vector and expressing it, the peptide can be produced in large quantities.

Meanwhile, the present invention provides a cosmetic composition comprising the peptide or the polynucleotide.

The cosmetic composition may be used for moisturizing, wrinkle improvement, and whitening.

The cosmetic composition of the present invention may include ingredients commonly used in cosmetic compositions, for example, conventional additives such as antioxidants, stabilizers, solubilizers, vitamins, pigments, and fragrances, as well as carriers.

The cosmetic composition of the present invention can be prepared in any formulation conventionally manufactured in the field, for example, in the form of a solution, suspension, emulsion, paste, gel, cream, lotion, powder, soap, surfactant-containing cleansing formulation, oil, powder foundation, emulsion foundation, wax foundation, or spray, but is not limited thereto.

More specifically, it can be formulated as a softening toner, nourishing toner, nourishing cream, massage cream, essence, eye cream, cleansing cream, cleansing foam, cleansing water, mask pack, spray, or powder.

When the formulation of the present invention is a paste, cream, or gel, the carrier component may be selected from animal oils, vegetable oils, waxes, paraffin, starch, tragacanth, cellulose derivatives, polyethylene glycol, silicone, bentonite, silica, talc, or zinc oxide.

When the formulation of the present invention is a powder or spray, the carrier component may be lactose, talc, silica, aluminum hydroxide, calcium silicate, or polyamide powder. In particular, when the formulation is a spray, it may additionally include a propellant such as chlorofluorohydrocarbon, propane/butane, or dimethyl ether.

When the formulation of the present invention is a solution or emulsion, the carrier component may be a solvent, solubilizer, or emulsifier, for example, water, ethanol, isopropanol, ethyl carbonate, ethyl acetate, benzyl alcohol, benzyl benzoate, propylene glycol, 1,3-butylene glycol oil, glycerol aliphatic esters, polyethylene glycol, or sorbitan fatty acid esters.

When the formulation of the present invention is a suspension, the carrier component may be a liquid diluent such as water, ethanol, or propylene glycol, a suspending agent such as ethoxylated isostearyl alcohol, polyoxyethylene sorbitol esters, or polyoxyethylene sorbitan esters, and microcrystalline cellulose, aluminum metahydroxide, bentonite, agar, or tragacanth.

When the formulation of the present invention is a surfactant-containing cleansing formulation, the carrier component may be selected from aliphatic alcohol sulfates, aliphatic alcohol ether sulfates, sulfosuccinate monoesters, isethionates, imidazolinium derivatives, methyltaurates, sarcosinates, fatty acid amide ether sulfates, alkylamidobetaines, aliphatic alcohols, fatty acid glycerides, fatty acid diethanolamides, vegetable oils, lanolin derivatives, or ethoxylated glycerol fatty acid esters.

When the cosmetic composition of the present invention is in the form of soap, a surfactant-containing cleansing formulation, or a surfactant-free cleansing formulation, it may be wiped off, peeled off, or rinsed with water after being applied to the skin. As a specific example, the soap may be liquid soap, powdered soap, solid soap, or oil-based soap; the surfactant-containing cleansing formulation may be a cleansing foam, cleansing water, cleansing wipe, or cleansing pack; and the surfactant-free cleansing formulation may be a cleansing cream, cleansing lotion, cleansing water, or cleansing gel, without being limited thereto.

Hereinafter, the present invention will be described in more detail with reference to Examples and Experimental Examples. However, the embodiments according to the present invention may be modified in various other forms, and the scope of the present invention should not be interpreted as being limited to the Examples described below. The Examples of the present invention are provided to more completely explain the invention to those skilled in the art.

### Example: Preparation of Peptide

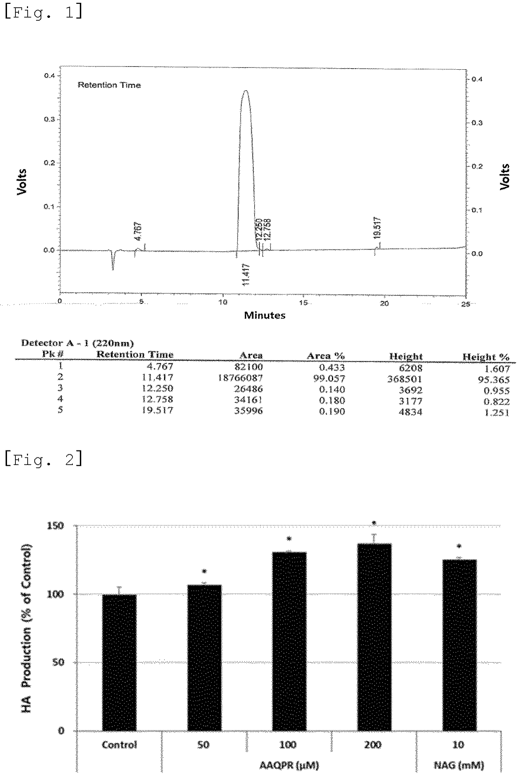

A peptide having the amino acid sequence of SEQ ID NO: 1, AAQPR (Ala-Ala-Gln-Pro-Arg), was synthesized using an automated peptide synthesizer (PeptrEX, Peptron, Republic of Korea). The synthesized peptide was confirmed to have 95% purity using C₁₈ reverse-phase high-performance liquid chromatography (HPLC, SHIMADZU Prominence, Japan), and the molecular weight (441) was verified by mass analysis (SHIMADZU LCMS-2020). The results are shown in Fig. 1.

For the above analysis, Vydac Grace smart RP C18 (4.6 X250 mm, 5 µm, Grace, USA) was used. The samples and standards were dissolved in water to a concentration of 1 mg/mL. The mobile phase consisted of distilled water containing 0.1% trifluoroacetic acid (TFA) and acetonitrile containing 0.1% trifluoroacetic acid (TFA). The flow rate was 1.0 mL/min, and detection was performed using a UV spectrophotometer at 220 nm. Elution was performed in gradient mode, with the proportion of acetonitrile containing 0.1% trifluoroacetic acid (TFA) gradually increased over 17 minutes.

### Experimental Example 1: Cell Culture

### Macrophage Culture

RAW264.7 macrophage cells were obtained from the American Type Culture Collection (ATCC) and cultured in Dulbecco's Modified Eagle's Medium (DMEM) containing 100 units/mL penicillin-streptomycin and 10% (v/v) fetal bovine serum (FBS) at 37°C in a 5% CO₂ incubator, and subculture was performed every 2 to 3 days.

### Keratinocyte Culture

The HaCaT human keratinocyte cell line was obtained from Cell Line Service GmbH (Germany). The cells were cultured in DMEM containing 1% penicillin-streptomycin and 10% fetal bovine serum (FBS) at 37°C in a 5% CO₂ incubator, and subculture was performed every 2 to 3 days.

### Dermal Fibroblast Culture

Normal Human Dermal Fibroblasts (NHDF) were obtained from Lonza (Lonza Walkersville, Inc.) and cultured in FBM (Fibroblast Basal Medium) supplemented with 0.1% hFGF-B, insulin, GA-1000, and 2% FBS at 37°C in a 5% CO₂ incubator. Subculture was performed every 2 to 3 days.

### Melanocyte Culture

Mouse-derived melanocytes (B16F10 cells) were obtained from the American Type Culture Collection (ATCC) and used. The cells were cultured in Dulbecco's Modified Eagle's Medium (DMEM) supplemented with 1% penicillin-streptomycin and 10% fetal bovine serum (FBS) at 37 °C in a 5% CO₂ incubator, and subcultured every 3 to 4 days.

### Experimental Example 2: Cytotoxicity Evaluation

### EZ-cytox assay

The EZ-cytox assay is a representative method for measuring cell viability using the principle that water-soluble tetrazolium salts (WSTs) react with dehydrogenases of viable cells to produce a water-soluble orange formazan.

### Cytotoxicity Evaluation in Macrophages

To evaluate cytotoxicity, RAW264.7 cells were seeded at 1.5 × 10⁴ cells/well in a 96-well plate using DMEM supplemented with 10% FBS and incubated for 18 hours at 37°C in a 5% CO₂ incubator. The test samples were then applied to the cultured cells. After treatment, EZ-cytox was added to each well and incubated for 30 minutes under the same conditions. Absorbance was measured at 450 nm using a microplate reader. The mean absorbance values of each sample group were compared with the control group to determine cell viability. No cytotoxicity was observed at the tested concentrations.

### Cytotoxicity Evaluation in Keratinocytes

HaCaT cells were seeded at 1.0-1.5 × 10⁴ cells/well in a 96-well plate using DMEM supplemented with 10% FBS and incubated for 24 hours at 37°C in a 5% CO₂ incubator. The medium was then replaced with serum-free DMEM, and the test samples were applied. EZ-cytox was added to each well and incubated for 30 minutes under the same conditions. Absorbance was measured at 450 nm using a microplate reader. The mean absorbance values of each sample group were compared with the control group to determine cell viability. No cytotoxicity was observed at the tested concentrations.

### Cytotoxicity Evaluation in Dermal Fibroblasts

NHDF cells were seeded at 1.0 × 10⁴ cells/well in a 96-well plate and incubated for 24 hours at 37°C in a 5% CO₂ incubator. The medium was then replaced with serum-free FBM, and the test samples were applied. EZ-cytox was added to each well and incubated for 30 minutes under the same conditions. Absorbance was measured at 450 nm using a microplate reader. The mean absorbance values of each sample group were compared with the control group to determine cell viability. No cytotoxicity was observed at the tested concentrations.

### Cytotoxicity Assessment on Melanocytes

To evaluate cytotoxicity, B16F10 cells were seeded at 2.5 × 10³ cells/well in a 96-well plate using DMEM supplemented with 10% FBS and cultured for 24 hours at 37 °C in a 5% CO₂ incubator. The cultured cells were then replaced with phenol red-free, serum-free DMEM, and the test samples were applied. EZ-Cytox was added to each well and incubated for 30 minutes at 37 °C in a 5% CO₂ atmosphere. Absorbance was measured at 450 nm using a microplate reader. The mean absorbance value for each sample group was calculated and compared with that of the control group to determine cell viability. No cytotoxicity was observed at the concentrations tested.

### Experimental Example 3: Verification of the Effect of Increasing Production of the Moisturizing Factor Hyaluronic Acid (HA)

HaCaT cells were seeded at 1.0 × 10⁵ cells/well in a 24-well plate and incubated for 24 hours at 37°C in a 5% CO₂ incubator. After incubation, the culture medium was replaced with serum-free DMEM, and the peptide prepared in the above Example was applied at concentrations of 50-200 µM, followed by incubation for 24 hours. The supernatant from each experimental group was collected, and the amount of hyaluronic acid (HA) released into the medium was measured. The positive control group was treated with N-acetylglucosamine at a concentration of 10 mM. Measurement of hyaluronic acid production was conducted using a Hyaluronic Acid ELISA Kit (Cusabio Biotechnology Co., Ltd.) following the manufacturer's protocol. The results of hyaluronic acid production following treatment with the peptide of the Example are shown in Table 1 and Fig. 2.

**[Table 1]**

| Concentration | | Hyaluronic acid production (%) |
|---|---|---|
| Untreated group | | 100±5.15 |
| AAQPR (µM) | 50 | 107.14±1.08 |
| | 100 | 130.93±0.59 |
| | 200 | 136.91±6.87 |
| NAG (mM) | 10 | 125.52±1.18 |

As shown in Table 1 and Fig. 2, treatment with the peptide of the Example resulted in an increase in hyaluronic acid production, confirming that the peptide of the present invention exhibits an excellent moisturizing effect.

### Experimental Example 4: Verification of Procollagen Type I Peptide (PIP) Production as a Wrinkle-Improving Factor

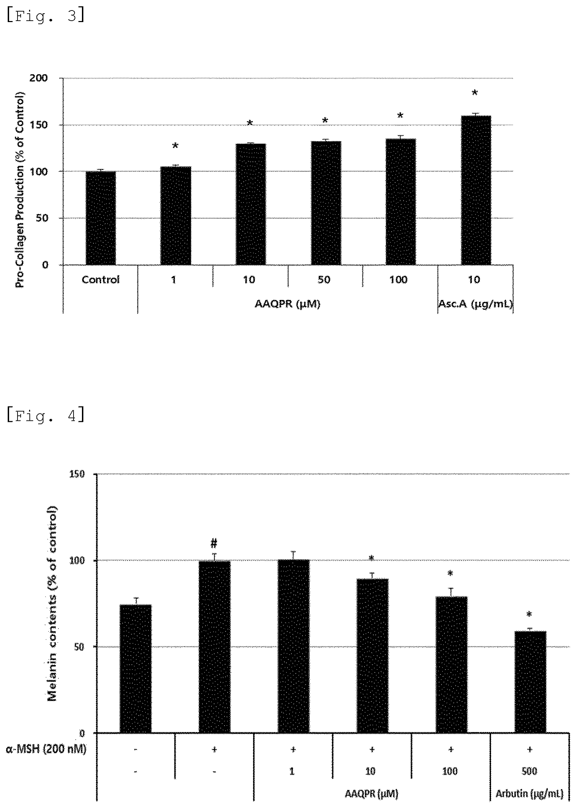

NHDF cells were seeded at 2.0 × 10⁴ cells/well in a 24-well plate and incubated for 24 hours at 37°C in a 5% CO₂ incubator. After incubation, the culture medium was replaced with serum-free FBM, and the peptide prepared in the above Example was applied at concentrations of 1-100 µM, followed by incubation for 24 hours. The supernatant from each experimental group was collected, and the amount of procollagen released into the medium was measured. The positive control group was treated with ascorbic acid at a concentration of 0.001 v/v%. Measurement of procollagen levels was performed using a Procollagen Type I Peptide (PIP) EIA Kit (Takara Biomedical Co.) according to the manufacturer's protocol. The quantitative results of procollagen type I peptide production following treatment with the peptide of the Example are shown in Table 2 and Fig. 3.

**[Table 2]**

| Concentration | | Procollagen production (%) |
|---|---|---|
| Untreated group | | 100±1.29 |
| AAQPR (µM) | 1 | 105.45±3.15 |
| | 10 | 129.75±9.74 |
| | 50 | 132.64±13.74 |
| | 100 | 134.92±1.3 |
| Asc.A (µg/mL) | 10 | 159.45±3.8 |

As shown in Table 2 and Fig. 3, treatment with the peptide of the Example resulted in an increase in procollagen production, confirming that the peptide of the present invention exhibits excellent wrinkle-improving and anti-aging effects.

### Experimental Example 5: Evaluation of Melanin Synthesis Inhibition by Whitening Factors

B16F10 cells were seeded at 6.0 × 10⁴ cells/well in a 6-well plate and cultured for 24 hours at 37 °C in a 5% CO₂ incubator. The culture medium was then replaced with phenol red-free, serum-free DMEM, followed by treatment with α-MSH, a melanin production-inducing hormone, and the peptide from the above Preparation Example at concentrations ranging from 1 to 100 µM. The cells were incubated for 72 hours, after which they were collected and lysed with 1 N NaOH in an 80 °C heating block to dissolve intracellular melanin. The absorbance of the resulting solution was measured at 490 nm. The control group was treated with arbutin at a concentration of 500 µg/mL. The results of melanin synthesis inhibition following treatment with the Preparation Example are shown in Table 3 and Figure 4.

**[Table 3]**

| Concentration | | α-MSH (200 nM) | Melanin production (%) |
|---|---|---|---|
| Untreated group | | | 75±3.04 |
| - | | + | 100±3.6 |
| AAQPR (µM) | 1 | + | 100.86±4.32 |
| | 10 | + | 89.62±2.95 |
| | 100 | + | 79.76±4.18 |
| Arbutin (µg/mL) | 500 | + | 59.6±0.8 |

As shown in Table 3 and Figure 4, treatment with the peptide of the Example inhibited melanin production, confirming that the peptide of the present invention exhibits an excellent whitening effect.

## Claims

1. An isolated peptide represented by the amino acid sequence of SEQ ID NO: 1.

2. A polynucleotide encoding the peptide of claim 1.

3. A cosmetic composition comprising the peptide of claim 1 or the polynucleotide of claim 2.

4. The cosmetic composition of claim 3, wherein the composition is a cosmetic composition exhibiting excellent moisturizing, wrinkle-improving and whitening effects.
